# EUROPEAN PATENT APPLICATION

(11) **EP 1 552 826 A1**
(43) Date of publication of application: **13.07.2005**
(21) Application number: 03748625.5
(22) Date of filing: 30.09.2003
(51) Int. Cl.: A61K 31/198, A61K 31/401, A61K 31/405, A61K 31/4172, A61P 25/28, A61P 43/00

(54) **AMINO ACID COMPOSITIONS FOR IMPROVING CENTRAL FUNCTIONS**

(30) Priority: 30.09.2002 JP 2002286487
(71) Applicant: Riken, Wako-shi, Saitama 351-0198 (JP); Meiji Dairies Corporation, Tokyo 136-0075 (JP); Abe, Takeshi, Warabi-shi, Saitama 335-0003 (JP)
(72) Inventor: ABE, Takashi, Warabi-shi, Saitama 335-0003 (JP); TSUCHITA, Hiroshi, Nishi-tokyo-shi, Tokyo 202-0015 (JP)
(74) Representative: Pätzold, Herbert, Dr.-Ing.
(86) International application number: PCT/JP2003/012532
(87) International publication number: WO 2004/028528

(57) **Abstract**

Amino acid composition or amino acid solution for improving central nervous functions containing threonine, proline, glycine, valine, isoleucine, leucine, tyrosine, phenylalanine, lysine, aspartic acid, serine, glutamic acid, alanine, methionine, tryptophan, histidine and arginine, and amino acid composition or amino acid solution derived from the above composition by removing tryptophan. These amino acid compositions have an action of improving the central nervous functions such as restoring of the central nervous, are nature-oriented, and are substantially free from adverse effects.

## Description

### TECHNICAL FIELD

The present invention relates to amino acid composition and amino acid solution having remarkable function with respect to central nervous function improvement, and more specifically to the amino acid composition and the amino acid solution which is contained in saliva secreted from the larva of a hornet (*Vespa* genus) and can be administrated for restoring the central nervous functions following fatigue.

### BACKGROUND OF INVENTION

The present inventors have investigated saliva secreted from the larva of a hornet, and have examined the use thereof together with clarification of the formulation of the amino acid composition contained therein.

As a result, the amino acid composition named with VAAM among a plenty of the amino acid compositions contained in the above saliva has been found to have a function of accentuating motor functions (Japanese patent No.2518692). The function of accentuating motor functions include muscle endurance, alimentation and tonicity, and fatigue restoration.

Serotonin, one of the physiologically active amines, is abundantly contained in enterochromafin cells in brain, pineal corpus, blood platelets, and bowels, and takes an important role in controlling the brain functions. The serotonin concentration in the brain is in close relation with the brain functions. The serotonin in the higher concentration is known to induce tiredness and hyprosis generation and to be extremely reduced by means of the interaction with dopamine or any other amine in brain in morbid dependence. Of course, the serotonin strongly functions for controlling emotion and body.

The serotonin in brain during exercise of a human is suggested to increase with increase of tryptophan due to exercise. This can be also conjectured from increase of tiredness, and induction of drowsiness and malaise. Such the central nervous functions following fatigue together with muscle fatigue is considered to a main factor for preventing the improvement of performance of exercise.

### DISCLOSURE OF THE INVENTION

An object of the present invention is to provide amino acid composition or amino acid solution which restores the central nervous functions following fatigue and can achieve the improvement of the central nervous functions.

The present invention is amino acid composition for improving central nervous functions comprising threonine, proline, glycine, valine, isoleucine, leucine, tyrosine, phenylalanine, lysine, aspartic acid, serine, glutamic acid, alanine, methionine, tryptophan, histidine and arginine. Further, amino acid solution, especially, aqueous solution having the same formulation as that of the above composition in a liquid phase is also included. As described later, the respective amino acids in the liquid phase have specified and preferable blending rates. The amino acid composition and its solution are nature-oriented so as to be substantially free from adverse effects, and are higher in a function of reducing the concentration of the serotonin which is an index of central nervous functions following fatigue so that they are excellent in improving the central nervous functions.

Although the improvement of the central nervous functions is hardly realized by removal of one or more amino acids from the above amino acid composition, the amino acid composition and the amino acid solution removal of only tryptophan are exceptions and have more excellent improvement of the central nervous functions than the above amino acid composition or its solution. Desirably, this amino acid composition or its solution also contains preferable blending rate as described later.

The present invention will be hereinafter described in detail.

As described above, VAAM has the action of accentuating the motor functions with the complicated mechanism. The VAAM is supposed to increase the motor functions as a result of assembly of many functions such as accentuation of fat metabolism, elevation of kidney functions and renal functions and body temperature increase.

From this viewpoint, the elevation of the central nervous functions is suggested to be indispensable for the elevation of the motor functions.

Oh the other hand, the serotonin in brain is a cause of central nervous functions following fatigue, and drowsiness, malaise and tiredness are considered to be based on the increase of the serotonin concentration in brain.

The investigation of the functions of the VAAM in the central nerve during exercise based on the change of the serotonin amount revealed that the above-described amino acid composition which is one example of the VAAM largely contributed to the restoration of the central nervous functions following fatigue.

That is, the present inventors have found that the administration of the VAAM having the above-described specified formulation to an athlete with the increased serotonin concentration in blood due to the central nervous fatigue removes the tiredness by means of the decrease of the serotonin concentration, thereby reaching the present invention.

First amino acid composition in accordance with the present invention (hereinafter referred to as first invention) contains, as essential components, 17 amino acids, and the respective amino acids are especially desirable when they are contained in the specified formulation rates. That is, the respective amino acids are contained such that 2 to 15 moles of threonine, 4 to 30 moles of proline, 7 to 20 moles of glycine, 4 to 8 moles of valine, 3 to 9 moles of isoleucine, 2 to 12 moles of leucine, 1 to 9 moles of tyrosine, 0.5 to 5 moles of phenylalanine, 5 to 11 moles of lysine, 0.1 to 5 moles of aspartic acid, 0.1 to 5 moles of serine, 0.1 to 4 moles of glutamic acid, 0.1 to 12 moles of alanine, 0.1 to 5 moles of methionine, 0.1 to 5 moles of tryptophan, 0.1 to 5 moles of histidine and 0.1 to 5 moles of arginine are satisfied. In addition, amino acids other than above, water-soluble vitamins, acid such as citric acid or a slight amount of other additives may be contained.

Second amino acid composition in accordance with the present invention (hereinafter referred to as second invention) contains, as essential components, 16 amino acids with the specified blending rate excluding the tryptophan from the amino acid composition of the first invention, and the tryptophan-free amino acid composition has the action of improving the central nervous function the same as or better than that of the first invention. The respective amino acids are preferably L-amino acids.

The amino acid compositions of the first invention and the second inventions may be taken in the form of powder or may be taken as aqueous solution after the dissolution in water. Administration can be conducted through commonly used paths such as oral administration, rectum administration, injection and infusion administration.

In case of the oral administration, other than the administration of the composition itself, the composition can be used as powder medicine, a granulated agent, a tablet, a capsule or a troche, together with the medically permissible support, shaping agent or diluting agent. However, a longer period of time may be required to absorb the solid powder medicine and the tablet so that the oral administration of the composition itself is preferable. In this case, the administration as a solution with a suitable additive such as a salt including sodium chloride, a pH adjuster or a chelating agent can be used. After the addition of a suitable buffer or isotonic agent and dissolution into sterilized and distilled water, the composition may be used as an injection agent.

The timing of taking the composition is not especially restricted, and the composition may be taken at arbitrary timing before or after generation of central nervous functions following fatigue. Especially, the intake as drinkable preparation (for example, cold beverage, powdery drinkable beverage, drinks as a medicine for a purpose of alimentation and tonicity and nutrition supplement).

The composition of the present invention is extremely lowly toxic, and an amount of administration can be established in a significantly wider range. An amount of administration depends on an administration method and a target of use, and ordinarily, one dose is 0.5 to 5 g and is preferably 1 to 2g, and daily dose is 1 to 20 g, and preferably is 4 to 10 g. When administrated or taken as solution, the composition in about 0.5 to 10% in weight of solution may be administrated or taken by 10 to1000 ml per every dose, and preferably the composition in 1 to 4% in weight of solution may be administrated or taken by 100 to 400 ml per every dose.

As apparent from the Examples described later, the amino acid compositions of the first invention and the second invention have a remarkable action with respect to the central nervous functions following fatigue by reducing the serotonin concentration, and further the amino acid compositions are nature-oriented and have the lower toxicity so as to improve the central nervous functions extremely efficiently.

As described before, the amino acid composition of the present invention is desirably used in the form of the solution, especially, in the form of the aqueous solution. In this case, the composition of the first invention or the second invention may be dissolved into water as it is or the respective amino acids were separately dissolved into water and then the above formulation may be realized in the solution.

### BRIEF DESCRIPTION OF DRAWINGS

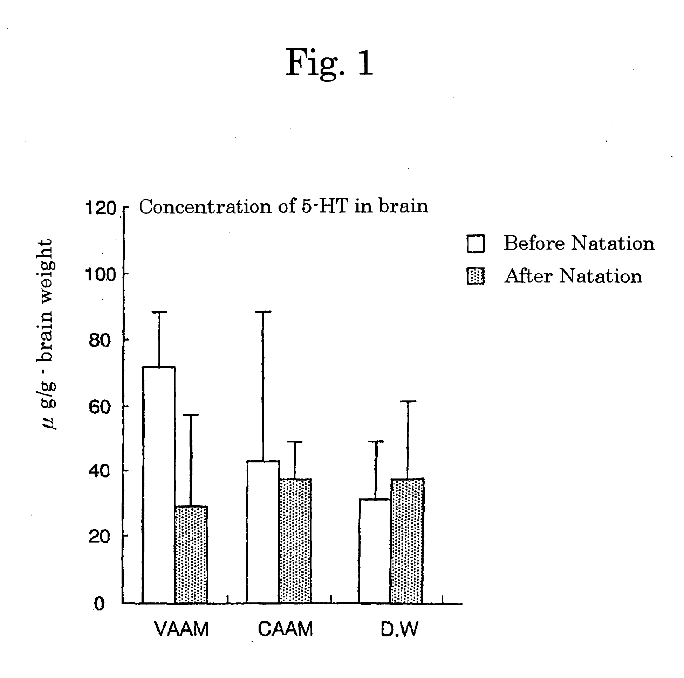

Fig.1 is a graph showing relations between VAAM, VAAM-TRP, CAAM or DW and serotonin concentrations.

### PREFERRED EMBODIMENTS OF IMPLEMENTING THE INVENTION

Although Examples and Comparative Examples regarding tests for improving central nervous functions of amino acid composition of the present invention will be described, the present invention shall not be restricted thereto.

### [Example 1]

### Preparation of VAAM

Nutrient solution containing composition of 17 amino acids shown in the column of Example 1 in Table 1 in a specified formulation was prepared by mixing commercially available amino acids and dissolving them into water.

### Exercise Test

After no food was supplied to ddy-type male mice (SPF) (five weeks from birth) (5 mice each group) for 12 hours in a clean room, 1.8% of the VAAM was orally administrated at a rate of 37.5µ L/g -body-weight. After 30 minutes' standing from the oral administration, blood was taken from five mice of the respective mice groups and the brains were taken out.

The remaining mice were attached with a 0.3g of a dead weight at the respective tails. Immediately after the natation for 30 minutes in a river pool at 35 °C, the blood was taken and the brains were surgically removed.

### Surgical Removal of Brains and Preparation of Sample

The weights of the brains surgically removed before and after the notation were immediately measured, and the brains were frozen with liquid nitrogen. To the frozen brain, an amount of 6% of PCA (perchloric acid) which was nine-times larger with respect to the brain, and 20 µL of BHT (dibutylhydroxytoluene) were added. After homogenization with Polytron homogenizer, it was centrifuged at 10,000 rpm for 10 minutes and at 4 °C by using a high speed centrifuging apparatus available from HITACHI, and 2 ml of supernatant liquid was taken and freeze-dried. To the dried sample, 100 µL of 0.1M acetic acid buffer (pH:4.5) was added and completely dissolved.

### Analysis of Serotonin

Absorption at 280 nm obtained by using a high speed chromatography available from Hitachi (L-6200) was measured by using an absorbance monitor available from Hitachi (L-4000UV-VIS) and recorded by using a chromatography pad available from Shimadzu Corporation. A column used was ODS-Silica (18c-5, 4.6 x 250 mm), and 0.1 mole of acetic acid buffer (pH:4.5) containing 10% methanol and 1mmol of EDTA (ethylenediamine tetraacetic acid) was used as a moving bed and a flow rate was 1.0 ml/cm. The results of the measurement were shown in a graph of Fig.1.

### [Comparative Examples 1 and 2]

In place of the VAAM of Example 1, CAAM (Comparative Example 1) or distilled water (Comparative Example 2, DW) shown in Table 1 were used. The compositions were administrated to mice under the same conditions as those of Example 1 and the serotonin concentrations were measured under the same conditions as those of Example 1. The results are shown in the graph of Fig.1.

**Table 1**

| Amino Acid (molar%) | Example | Compar. Example | |
|---|---|---|---|
| | 1 | 1 | 2 |
| | VAAM | CAAM | DW |
| Asp | 0.2 | 7.5 | - |
| Thr | 7.2 | 2.5 | - |
| Ser | 2.5 | 8,0 | - |
| Glu | 3.2 | 19.6 | - |
| Pro | 18.0 | 8.5 | - |
| Gly | 19.1 | 4.5 | - |
| Ala | 6.0 | 4.5 | - |
| Val | 5.9 | 5.5 | - |
| Cys | - | 0.4 | - |
| Met | 0.5 | 2.5 | - |
| Ile | 4.5 | 5.5 | - |
| Leu | 6.2 | 8.5 | - |
| Tyr | 6.0 | 5.0 | - |
| Phe | 3.8 | 4.0 | - |
| Lys | 8.6 | 7.0 | - |
| Trp | 2.2 | 1.0 | - |
| His | 2.6 | 2.5 | - |
| Arg | 3.5 | 3.0 | - |

### Investigation Regarding Example 1 and Comparative Examples 1 and 2

The serotonin concentration measured before the notation of the VAAM administrate-group of Example 1 was highest, that of the CAAM administrate-group of Comparative Example 1 was lower, and that of the DW administrate-group of Comparative Example 2 was lowest (refer to Fig.1, 5-HT in Fig.1 refers to serotonin).

The serotonin concentration measured after the notation of the CAAM administrate-group of Comparative Example 1 was highest, that of the DW administrate-group of Comparative Example 2 became higher, and that of the VAAM administrate-group of Example 1 was lowest (refer to Fig.1).

When the serotonin concentrations before and after the notation were compared, the concentration drop of the VAAM administrate-group of Example 1 was largest, that of the CAAM administrate-group of Comparative Example 1 became smaller, and that of the DW administrate-group of Comparative Example 2 was inversely increased.

While the serotonin in brain is said to be proportional to tryptophan concentration in blood, the serotonin concentration before the natation in the graph of Fig.1 is maximum. Although the tryptophan content in VAAM is twice or more as much as that in CAAM, the serotonin concentration in brain of the VAAM administrate-group after the natation was lower than the concentration of the CAAM administrate-group.

These results strongly suggest that the VAAM administrate-group has a special action on brains.

Since the above embodiments are described only for examples, the present invention is not limited to the above embodiments and various modifications or alterations can be easily made therefrom by those skilled in the art without departing from the scope of the present invention.

## Claims

1. Amino acid composition or amino acid solution for improving central nervous functions comprising threonine, proline, glycine, valine, isoleucine, leucine, tyrosine, phenylalanine, lysine, aspartic acid, serine, glutamic acid, alanine, methionine, tryptophan, histidine and arginine.

2. The amino acid composition or the amino acid solution for improving the central nervous functions as defined in claim 1, wherein the respective amino acids are contained such that 2 to 15 moles of threonine, 4 to 30 moles of proline, 7 to 20 moles of glycine, 4 to 8 moles of valine, 3 to 9 moles of isoleucine, 2 to 12 moles of leucine, 1 to 9 moles of tyrosine, 0.5 to 5 moles of phenylalanine, 5 to 11 moles of lysine, 0.1 to 5 moles of aspartic acid, 0.1 to 5 moles of serine, 0.1 to 4 moles of glutamic acid, 0.1 to 12 moles of alanine, 0.1 to 5 moles of methionine, 0.1 to 5 moles of tryptophan, 0.1 to 5 moles of histidine and 0.1 to 5 moles of arginine are satisfied.

3. Tryptophan-free amino acid composition or amino acid solution for improving central nervous functions comprising threonine, proline, glycine, valine, isoleucine, leucine, tyrosine, phenylalanine, lysine, aspartic acid, serine, glutamic acid, alanine, methionine, histidine and arginine.

4. The tryptophan-free amino acid composition or the amino acid solution for improving the central nervous functions as defined in claim 3, wherein the respective amino acids are contained such that 2 to 15 moles of threonine, 4 to 30 moles of proline, 7 to 20 moles of glycine, 4 to 8 moles of valine, 3 to 9 moles of isoleucine, 2 to 12 moles of leucine, 1 to 9 moles of tyrosine, 0.5 to 5 moles of phenylalanine, 5 to 11 moles of lysine, 0.1 to 5 moles of aspartic acid, 0.1 to 5 moles of serine, 0.1 to 4 moles of glutamic acid, 0.1 to 12 moles of alanine, 0.1 to 5 moles of methionine, 0.1 to50 moles of histidine and 0.1 to 5 moles of arginine are satisfied..
